# EUROPEAN PATENT APPLICATION

(11) **EP 4 502 140 A1**
(43) Date of publication of application: **05.02.2025**
(21) Application number: 23189180.5
(22) Date of filing: 02.08.2023
(51) Int. Cl.: C12N 1/20, C12R 1/46, A23C 9/12, A23L 33/135

(54) **LACTOCOCCUS CREMORIS STRAIN AND USE THEREOF FOR PREPARING FOOD PRODUCTS**

(71) Applicant: Sacco S.r.l., 22071 Cadorago (IT)
(72) Inventor: VOLONTÈ, Federica, Valmorea (IT); DAL BELLO, Fabio, Cirimido (IT); MARINILLI, Maurizio, Caslino al Piano (IT); CANNONE, Francesca, Albavilla (IT)
(74) Representative: Zaccaro, Elisabetta

(57) **Abstract**

The present invention concerns the field of food products and specifically relates to new strains of *Lactococcus cremoris* with improved properties.

The invention further relates to a composition comprising the *Lactococcus cremoris* strain, and to a food product, in particular a dairy product and plant based milk products prepared by using the strain. The invention is also extended to the application of the improved *Lactococcus cremoris* strains in the fermentation of vegetable and fruit matrices.

## Description

### FIELD OF THE INVENTION

The present invention relates to a novel lactic acid bacterium (LAB), namely a novel *Lactococcus cremoris* strain, which acquired improved texturizing properties. The present invention also relates to methods of using the strain as an ingredient for making food products and to food products comprising the strain as ingredient. The invention further relates to the combination of the improved *L. cremoris* strains with other LAB of the genera *Streptococcus, Lactococcus, Leuconostoc, Lactobacillus, Pediococcus* and *Bifidobacterium* and to a food, in particular a dairy or dairy-alternative fermented product, prepared using milk or plant based extracts and fermented by using the above improved strains.

### STATE OF THE ART

Lactic acid bacteria (LAB) are extensively used in the food industry to prepare foods by fermentation. LAB fermented products such as fermented milk, drinks and/or cheeses are characterized by unique sensory, texture, shelf life and overall quality. Carefully selected strains are combined into the formulation of food cultures that are used by the food industry as ingredients for the production of many different fermented foods.

The starter culture industry is selecting strains based on the characterization of their performance, i.e., acidification, reduced post-acidification, preservation, aroma development as well as texturizing properties. Good texture is typically high mouth thickness and viscosity (measured with viscosimeter at constant and increased share rate) and high gel firmness.

The contribution of LAB strains to improved texture is due to their ability to produce Exo-(or extracellular) PolySaccharides (EPS), which can be capsular (attached to the cell in the form of capsules) or secreted into the media. EPS-producing LAB are of interest, since EPS act as natural viscosifier and texture enhancer in fermented foods. EPS producers thus have the ability to positively influence viscosity, syneresis, firmness and overall sensory properties of the food product generated through their fermentation.

Traditional fermented milk products can be obtained by using mesophilic LAB, e.g., *Lactococcus* sp. , and/ or thermophilic LAB, e.g., *Streptococcus salivarius subsp. thermophilus.* Products such as sour milk and yoghurt are obtained using the first or second group of LAB, respectively. Recently, there has been a growing interest in the development and production of dairy-alternative products, where plant bases are fermented using LAB, among which also mesophilic starter cultures such as *Lactococcus cremoris* strains.

*L. cremoris* is a LAB and one of the most important microorganisms used by the dairy industry. It is a gram-positive bacterium, nonsporulating and are nonmotile. *L. cremoris* has a homofermentative metabolism, meaning lactic acid is the main metabolite produced from sugars. Some strains of L. cremoris are able to produce EPS and thus influence the overall texture of the products. These strains are commonly used for the production of fermented beverages such as buttermilk and other traditional North-East European products, as well as many different cheeses.

The number of EPS-producing *L. cremoris* strains found in nature is however limited, and so is the type and amount of EPS that can be produced during fermentation. Therefore, there is an increasing need to find improved *L. cremoris* strains, in order to ensure the production of fermented dairy products with improved qualities and texture.

### SUMMARY OF THE INVENTION

The authors of the present invention have surprisingly identified two *Lactococcus cremoris* strains, having improved qualities, when compared to the parental strain, for the production of fermented milk and cheese as well as plant based matrixes.

The invention therefore relates to *L. cremoris* strains, wherein said strains are:
- *L. cremoris* SC226, deposited with accession number DSM 34237 at the Leibniz Institute DSMZ under the Budapest Treaty on 23 February 2022;
- *L. cremoris* SC244, deposited with accession number DSM 34238 at the Leibniz Institute DSMZ under the Budapest Treaty on 23 February 2022

In a second aspect the present invention relates to a composition comprising one or more of the *L. cremoris* strain SC226 or the *L. cremoris* strain SC244, according to the invention.

In a third aspect the present invention relates to the use of one or more of the *L. cremoris* strain SC226 or the *L. cremoris* strain SC244 or of the composition comprising the same, for the preparation of a starter formulation.

In a fourth aspect the present invention relates to the use of one or more of the *L. cremoris* strain SC226 or the *L. cremoris* strain SC244 or of the composition comprising the same, for the preparation of a food product.

In a fifth aspect the present invention relates to a food or beverage product comprising one or more of the *L. cremoris* strain SC226 or the *L. cremoris* strain SC244, deposited respectively with accession number DSM 34237 and DSM 34238 at the DSMZ.

In a sixth aspect the present invention relates to a method for preparing a dairy product, a milk product, a plant-based product or a vegetal and/or fruit product, said method comprising the step of using one or more of the *L. cremoris* strain deposited with accession number DSM 34237 at the DSMZ or the *L. cremoris* strain deposited with accession number DSM 34238.

In a seventh aspect the present invention relates to a method to prepare fermented plant based milk or vegetable/fruit based fermented preparations, said method comprising the use of the *L. cremoris* strain deposited with accession number DSM 34237 at the DSMZ and/or *L. cremoris* strain deposited with accession number DSM 34238 at the DSMZ.

### BRIEF DESCRIPTION OF THE DRAWINGS

The characteristics and advantages of the present invention will be apparent from the detailed description reported below, from the Examples given for illustrative and non-limiting purposes, and from the annexed Figures 1-12, wherein:
Figure 1: acidification profile at different temperatures of the strains SC226 and SC244, in comparison with the parental strain SC96.
Figure 2: overall texture values measured with Ford cup (s/g, seconds/gram) achieved for milk cultures inoculated with liquid cultures of SC96, SC226 or SC244 strains and grown at 22 or 37°C.
Figure 3: viscosity measured with the viscosimeter at constant share rate (A) or with progressive increase of the shear rate (B) in milk inoculated with SC96 or SC226, fermented at different temperatures.
Figure 4: main results achieved in Trial 1. Acidification profiles and characteristics of the fermented product achieved in blends where SC96 has been replaced with SC226, incubated at 32 C° (A) or at 37 C° (B). In some of the trials the EPS producing strain ST386 (a strain of *Streptococcus salivarius* subsp. *thermophilus)* has been removed and the EPS contribution was given just by the mesophilic strains SC96 or the SC226 exclusively.
Figure 5: acidification profile and product characterization of blends created using SC96 or SC226.
Figure 6: results achieved at the viscosimeter with the blends created using SC96 or SC226: A), results achieved setting a constant shear rate; B), results obtained in a ramp test with falling curve.

### DETAILED DESCRIPTION OF THE INVENTION

In accordance with the above surprising findings, the present invention relates to two texturizing lactic acid bacteria strains, belonging to the species *Lactococcus cremoris.* Said strains are called SC226 and SC244 and are deposited with accession number DSM 34237 and DSM 34238, respectively, at the Leibniz Institute DSMZ under the Budapest Treaty on 23 February 2022.

The *L. cremoris* strains of the invention can be present in a culture, such as a starter culture. Advantageously, the inventors have found that the SC226 and SC244 strains have improved texturizing capacity when compared to the parental strain SC96. In fact, the viscosity of the resulting milk inoculated with the SC226 or SC244 cultures was seen to be optimal, with increased body and ropiness, and did not present clumps even when fermented at higher temperature (e.g., 37 °C), in contrast with the results obtained by using the parental strain SC96. The increased viscosity of the strains allows to reach the same performance of the parental strain, even with a reduced inoculum (more advantageous from an economic point of view). Further characterization in blends with other strains, after scaling up, were performed mostly with the strain SC226, combined with other strains belonging to other species. All the evidence of the improved properties of the *L. cremosis* strain SC226 in milk and plant-based extracts can be extended also to the strain SC244.

As can be seen from the results in Example 3, the acidification performance of the SC226 strain was maintained also with the scaling-up, and the viscosity was even increased, further highlighting the improvement of the strain SC226, with respect to the parental strain SC96.

Data collected in Example 4 demonstrate that SC226 is consistently given high quality products, also when reduced in the amount of the inoculum, with respect to the parental strain SC96: this aspect demonstrate that SC226 is economically advantageous.

In a second aspect the present invention relates to a composition comprising one or more of the the *L. cremoris* strain SC226 or the *L. cremoris* strain SC244, according to the invention.

The composition in one embodiment preferably comprises an amount of the *L. cremoris* strain SC226 strain or the *L. cremoris* strain SC244 to allow obtaining the desired acidifying and texturizing results.

Composition of the invention can be prepared with the SC226 strain or the SC244 strain, or with blends of the SC226 or SC244 and other LAB strains. As will be evident from the results in Example 4, SC226 works very well both alone and in blends, contributing both to the acidification profile as well as the overall texture.

In a preferred aspect the invention relates to the combination of the *L. cremoris* strains of the invention with other LAB of the genera such as for example strains of *Streptococcus, Lactococcus, Leuconostoc, Lactobacillus, Pediococcus* and *Bifidobacterium.*

In a preferred embodiment the *L. cremoris* strain is lyophilized, frozen pellet, heat-treated or in the form of a suspension. The composition may advantageously be usable as a starter culture, and can be in frozen, freeze-dried or in liquid form, for example in the form of a suspension. Preferably, the composition comprises milk, in which the SC226 strain allows to develop specific dairy products with interesting aromas.

As used herein, the term "lactic acid bacterium" designates a gram-positive microaerotollerant bacterium, which ferments sugars with the production of organic acids including lactic acid as the predominantly produced acid, acetic acid and propionic acid. The industrially most useful LAB are found within the order *"Lactobacillales"* which includes *Lactococcus* spp., and other lactic acid bacteria, including bacteria of the species *Lactobacillus* sp. and *Streptococcus salivarius* subsp. *thermophilus,* are normally supplied to the dairy industry either as frozen or freeze-dried cultures for bulk-set or direct-vat set (DVS) starter cultures production, which are used for direct inoculation into a fermentation vessel or vat for the production of a dairy product, such as a fermented milk product. Such cultures are in general referred to as "food cultures", "starter cultures" or "starters".

As used herein, the term "milk" refers to the lacteal secretion obtained by milking any mammal, such as cows, sheep, goats, buffaloes or camels. In a preferred embodiment, the milk is cow's milk. The term milk also includes protein/fat solutions made of plant materials, e.g., soy milk.

As used herein, "dairy products" refers to raw and processed or manufactured milk and milk-derived products. Non-limiting exemplary dairy products include plain yoghurt, flavoured yoghurt, fruit-yoghurt, flavoured milk drinks, fermented milk drinks with or without fruit preparation or flavouring, hard cheese or soft cheese, ice cream, cream or cream-based desserts.

In a third aspect the present invention relates to the use of one or more of the *L. cremoris* strain SC226 or the *L. cremoris* strain SC244 or of the composition comprising the same, for the preparation of a starter formulation.

In a fourth aspect the present invention relates to the use of one or more of the *L. cremoris* strain SC226 or the *L. cremoris* strain SC244 or of the composition comprising the same, for the preparation of a food product.

In a fifth aspect the present invention relates to a food or beverage product comprising one or more of the *L. cremoris* strain SC226 or the *L. cremoris* strain SC244, deposited with accession numbers DSM 34237 and DSM 34238 at the DSMZ.

In a preferred embodiment, the food product is a dairy product or a milk product, a plant based product or a vegetable and/or fruit product. Preferably the food product can be a fermented product, for example a fermented milk product, fermented with the cultures of the *L.cremoris* strain SC226.

In a sixth aspect the present invention relates to a method for preparing a dairy product or a milk product, a plant-based product or a vegetal and/or fruit product, said method comprising the step of using one or more of the the *L. cremoris* strains deposited with accession numbers DSM 34237 or DSM 34238 at the DSMZ.

In a seventh aspect the present invention relates to a method to prepare fermented plant based milk or vegetable/fruit based fermented preparations, said method comprising the use of the *L. cremoris* strain deposited with accession number DSM 34237 at the DSMZ and/or *L. cremoris* strain deposited with accession number DSM 34238 at the DSMZ.Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention.

### Example 1.: selection of strains SC226 and SC244

The strain according to the present invention was obtained after a conjugation trial between SC96 (L. *cremoris)* strain and SL56 *(Lactococcus lactis* subsp. *lactis)* in presence of 10⁷ pfu/ml of the bacteriophage 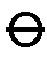 SL56. A strict selection condition was applied: serial dilutions were plated on milk plates containing 0.1 µg/ml of ampicillin at 40°C.

The resulting colonies were inoculated in milk and incubated for 18 h at 30°C. The milk cultures were assayed for exopolysaccharides (EPS) production: the ropy ones were selected for further characterization.

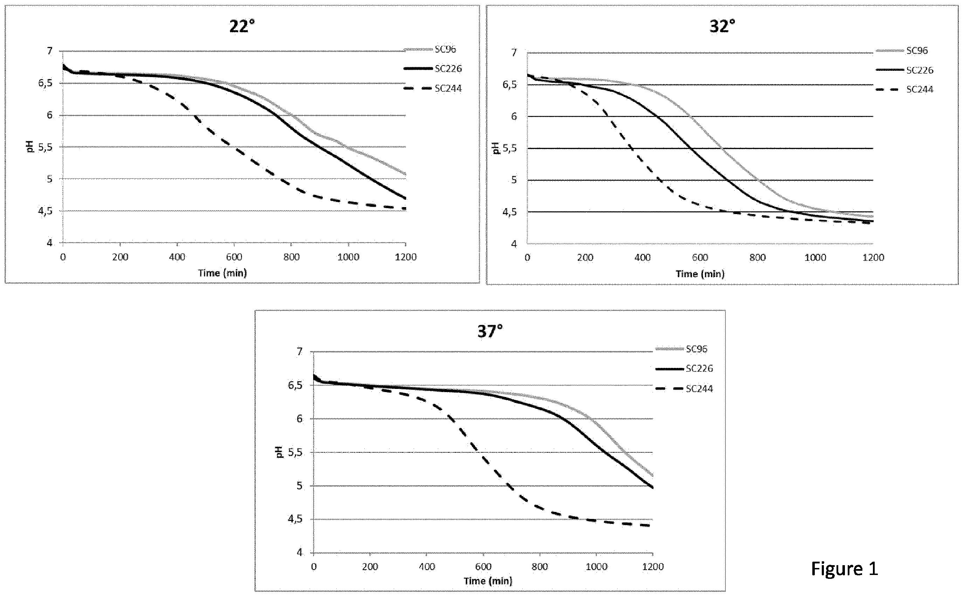

SC226 and SC244 were classified as *Lactococcus cremoris* strains with improved texturizing properties when compared to the parental strains SC96. The SC226 and SC244 strains milk acidification profiles at different temperatures were checked in comparison to SC96, after 2% inoculum in pasteurized milk (Figure 1). The capability to acidify milk has been kept by the strains SC226 and SC244, which resulted even slightly faster than the parental SC96 in the milk acidification at different growth temperatures, especially the higher ones.

### Example 2.: SC226 and SC244 strains characterization

The EPS synthesis was achieved at all the tested temperatures and clearly visible also in MRS broth.

SC226 and SC244 resulted both significantly improved in texturizing capacity, according to the achieved texture values (s/g) measured in milk.

The texture of the resulting milk inoculated with the SC226 or SC244 cultures was measured with a Ford cup: pasteurized milk was 1 % inoculated with a milk culture of SC226 or SC244 and incubated at 22°C or 37°C. Once a pH of 4.5 was reached, the coagulum was broken, refrigerated and stored overnight at 4°C.

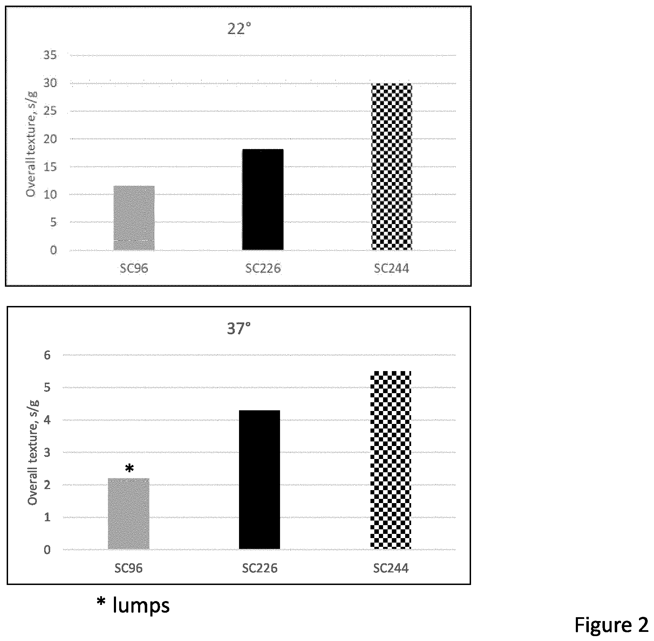

The next day, the coagulum was broken again and the overall texture was measured (Figure 2). In general, with both SC226 and SC244 were achieved values of texture almost or more than doubled, both at mesophilc temperatures and high temperatures, with respect to the parental SC96. Furthermore, as can be seen in Figure 2, lumps were also present in the milk preparation obtained with SC96 at 37°C, while the milk prepared with SC226 or SC244 did not have this defect.

The same milk fermentation set up at different temperature were also characterized using a viscosimeter (rotational viscometer first prodig CP1000) for the strain SC226.

The analysis were conducted using the CP6020 spin (60mm diameter, 20° angle). Both the temperature of the sample and the thermostatation of the plate was 10°C.

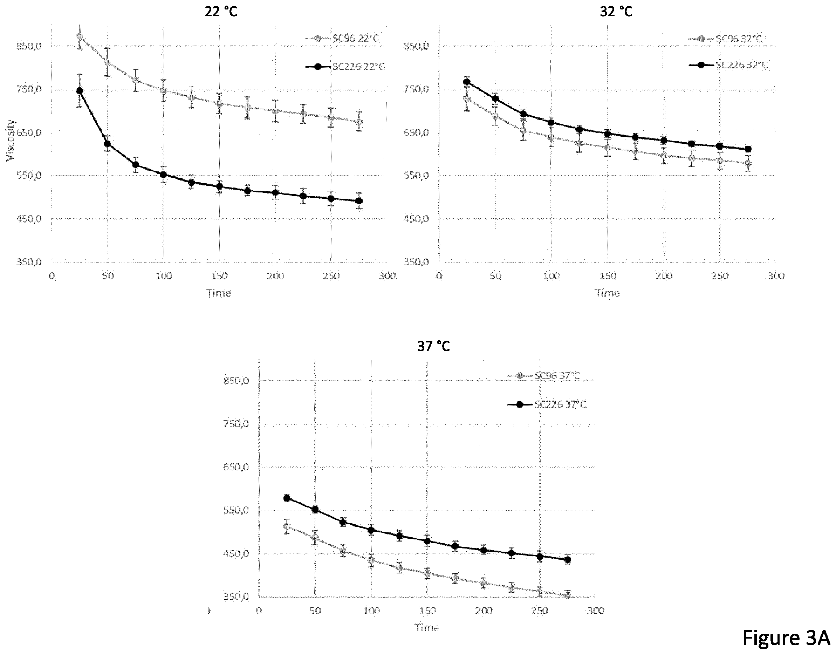

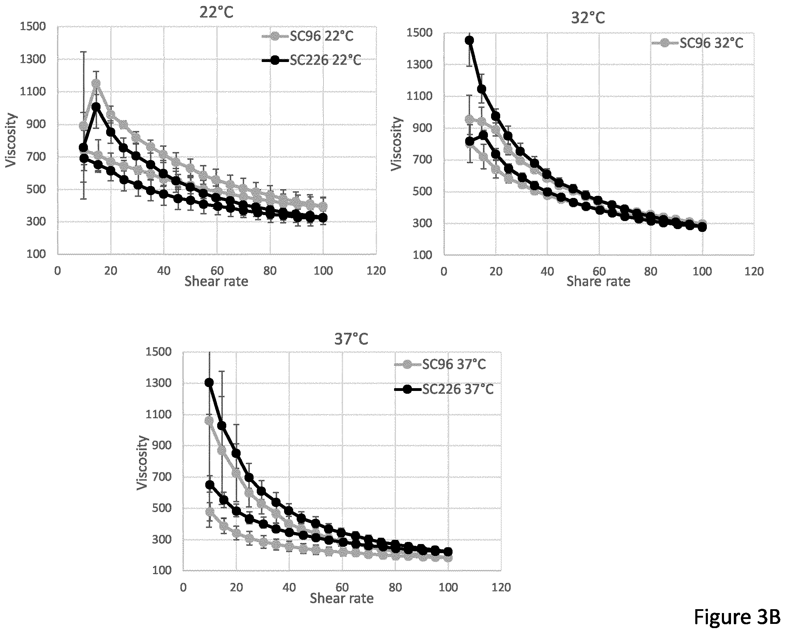

Two different program were used in order to measure the viscosity of the samples. The first with a constant shear rate value (30s-1) and measuring point every 25 seconds (Figure 3A), the second with an increasing value of shear rate and falling curve (Figure 3B).

Prior to viscosity measurements, the yogurts were manually stirred according to a standardized protocol. Also, the time between homogenization and viscosity measurement was standardized for all the samples.

At 22 °C SC96 resulted more viscous, even if the overall texture measured in Ford cup resulted significantly lower with the parental strain. The texture achieved with the improved strain SC226 is possibly such increased that could be underestimated through the viscosimeter because of slippering effect. At the higher temperature, especially at 37°C (not typical for mesophilic culture) anyway instead, SC226 is significantly more viscous than the parental SC96. Notably, in fermented product prepared at 37°C, SC226 creates a structure more resistant to shear stress: there is a lower loss in viscosity in trials at constant shear rate (Figure 3A) and the return curves of the rap trials (Figure 3B) are closer to the forward one for SC226 with respect to the parental SC96.

### Example 3.: SC226 scale-up

SC226 was produced on an industrial scale till 20000 liters as frozen and freeze-dried products. The acidification performance of the strain according to the invention was maintained with the scaling-up and the overall texture was even increased (with respect to the results achieved with the liquid cultures), further highlighting the improvement of the strain SC226, with respect to the parental SC96 (Table 1).

**Table 1: average of the texture values (s/g) at 22°C achieved with both frozen and freeze-dried cultures from production.**

| **Strain** | **Freeze-dried** | **Frozen** |
|---|---|---|
| SC96 strain (parental) | 7.7 | 8.4 |
| SC226 strain | 17.9 | 27.0 |

### Example 4.: SC226 blends

The industrial products have been used in blends with other strains, also belonging to different species.

### Trial 1

Blend containing strains of *Lactococcus cremoris* and *Streptococcus salivarius thermophilus.*

Previous blends (with and without *Streptococcus salivarius subsp. thermophilus* strain ST386) which contained the parental strain SC96, were compared to those in which SC226 replaced SC96.

Specifically, two different evaluations of sensorial analysis were made:
- The substitution of the parental strain SC96 with SC226 in the blend; and
- The removal of the strain of *Streptococcus salivarius subsp. thermophilus* ST386, which contributes to the overall texture.

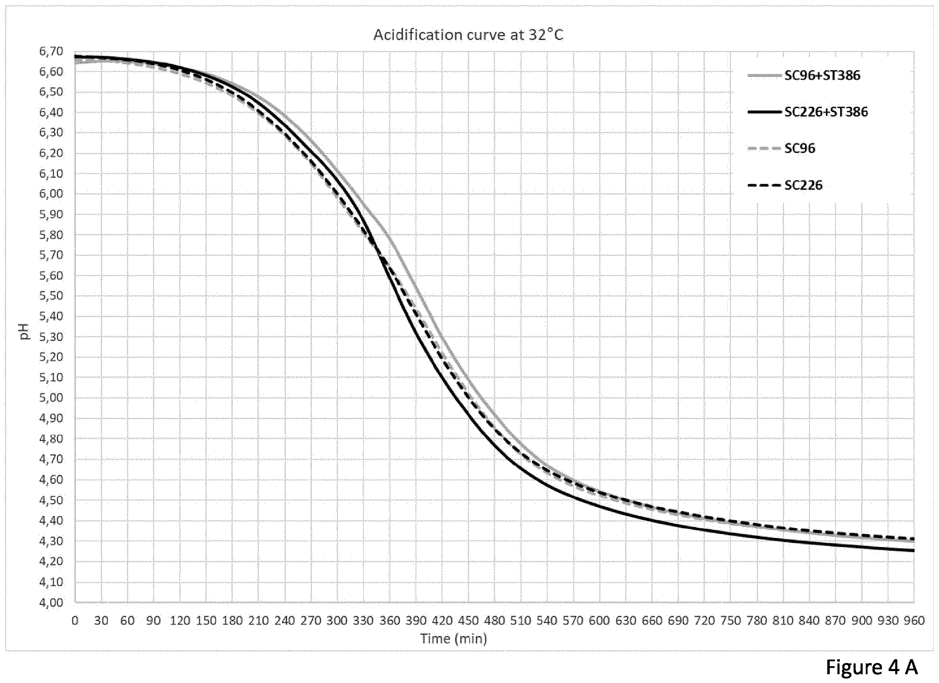

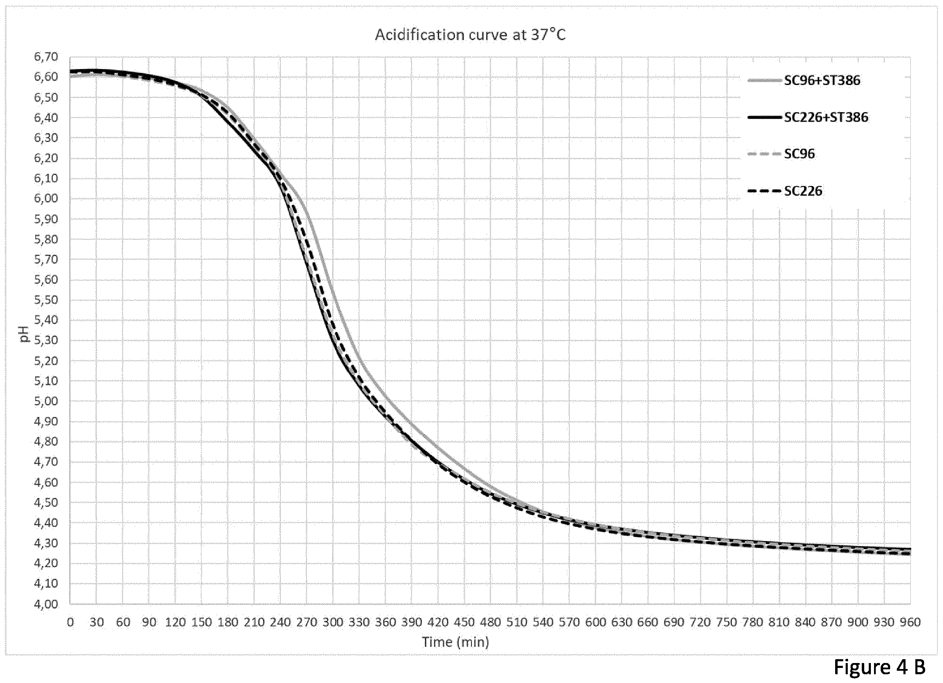

The trials have been set up both at 32 and 37°C (Figure 4, A and B).

The acidification at 32 C° is improved for the blend containing SC226 with respect to the one with the parental SC96 (see gray line, Figure 4A).

In addition, as can be seen in Table 2, the overall texture of the blend is increased to 5.4 s/g with SC226 and furthermore significantly increased to 7 s/g when the viscous strain of *Streptococcus salivarius subsp. thermophilus* ST386 is removed from the blend.

**Table 2:**

| **32°C** | **Day after** | | **Sensory analysis Stirred** | | | | | |
|---|---|---|---|---|---|---|---|---|
| | **pH** | **Overall texture (s/g)** | **Ropiness** | **Body** | **Lumpiness** | **Acidity** | **Aroma** | **Gas** |
| SC96+ST386 | 4,34 | **4,9** | **II/III** | **II+** | 0+ | I+ | I+ | 0 |
| **SC226+ST386** | 4,40 | **5,4** | **II/III** | **II** | 0 | I | I+ | 0 |
| SC96 | 4,42 | **3,0** | **I+** | **I+** | 0 | I | I | 0 |
| **SC226** | 4,37 | **7** | **II/III** | **II+** | 0 | I+ | I | 0 |

The SC226 strain, according to the invention, thus can significantly contribute to the product viscosity, both with an additional strain and when the strain is the only EPS producing strain in the blend.

The same behavior has been interestingly confirmed also at 37° C, even if *Lactococcus cremoris* species is mesophilic (Figure 4B and Table 3).

**Table 3:**

| **37°C** | **Day after** | | **Sensory analysis Stirred** | | | | | |
|---|---|---|---|---|---|---|---|---|
| | **pH** | **Overall texture (s/g)** | **Ropiness** | **Body** | **Lumpiness** | **Acidity** | **Aroma** | **Gas** |
| SC96+ST386 | 4,42 | **1,9** | **0/I** | **I+** | 0 | I | I+ | 0 |
| **SC226+ST386** | 4,42 | **6,4** | **II** | **I/II** | 0 | I | I | 0 |
| SC96 | 4,39 | **<1** | **0** | **0+** | III- | I | I | 0 |
| **SC226** | 4,44 | **1,2** | **0** | **0/1** | II+ | I | I | 0 |

The contribution of the SC226 strain can be observed both in the acidification profile and in the overall texture: the removal of the viscous thermophilic strain ST386 (belonging to the species *Streptococcus salivarius subsp. thermophilus)* and the substitution with SC226 as the only EPS producing strain leads to faster acidification profiles and dairy products which are less lumpy and more viscous (with respect to the counterpart set up with the parental strain SC96 as only EPS producer) (Figure 4B and Table 3).

The *Lactococcus cremoris* strains SC226 or SC244 were used as ingredients together with *Streptococcus salivarius subsp. thermophilus* strains to ferment plant based matrices (mostly soy and aot): they can grow in these substrates, improving the texture of the obtained food products and beverages.

### Trial 2:

Blend containing other strains of *Lactococcus cremoris, Lactococcus lactis* subsp. *lactis biovar diacetylactis* and *Leuconostoc mesenteroides* strains, suitable for set products.

The improvement in the overall structure of the blend by using SC226 instead of its parental SC96 has been reported, both at 22° C and 32° C (Table 4).

**Table 4A and 4B: results of the panel test on products obtained using mesophilic bends, using the strains SC226 or the parental strain SC96.**

| **A** | **Day after** | | **Sensory analysis Stirred** | | | | | |
|---|---|---|---|---|---|---|---|---|
| 22°C | **pH** | **Overall texture (s/g)** | **Ropiness** | **Body** | **Lumpiness** | **Acidity** | **Aroma** | **Gas** |
| SC96 | 4,42 | **3,5** | **II+** | **I+** | 0 | I | I | 0+ |
| **SC226** | 4,39 | **5,5** | **III** | **II/III** | 0 | I | I/II | 0 |
| | | | | | | | | |

| **B** | **Day after** | | **Sensory analysis Stirred** | | | | | |
|---|---|---|---|---|---|---|---|---|
| 32°C | **pH** | **Overall texture (s/g)** | **Ropiness** | **Body** | **Lumpiness** | **Acidity** | **Aroma** | **Gas** |
| SC96 | 4,26 | **1,3** | **0** | **0/I** | I/II | I | II | 0+ |
| **SC226** | 4,27 | **2,5** | **I** | **I** | 0+ | I | I/II | 0 |

Also, for this product, the use of SC226 lead to an increased viscosity and to the improvement of desired features.

In fact, as can be seen in the results shown in Table 4, the SC226 strain showed a higher viscosity both at 22°C and 32°C. The addition of SC226 instead of SC96 resulted in a product with typical aroma, reduced lumpiness and significantly improved in texture (considering both body and ropiness).

### Trial 3:

The SC226 products were used in other blends with *Lactococcus cremoris, Lactococcus lactis* subsp. *lactis biovar diacetylactis* and *Leuconostoc mesenteroides,* these blends are useful to develop dairy products with specific aromas.

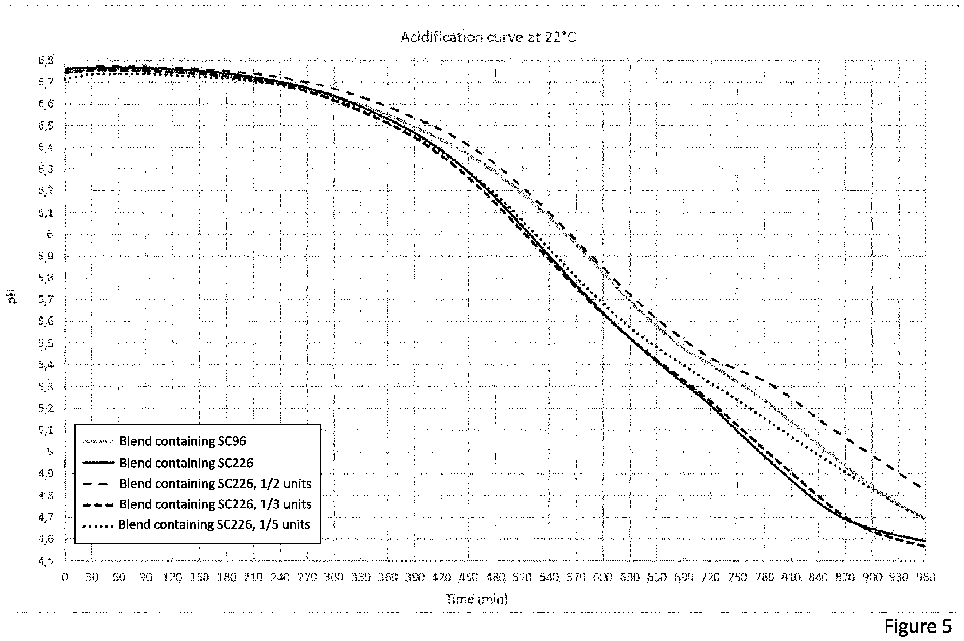

The replacement of the parental strain SC96 with the new SC226 strain led to the development of a dairy product with an optimal texture and a good aroma. The overall texture was 13 s/g, which is higher than the comparative product set up with SC96. Similar texture values were reached also reducing the SC226 amount at, 1/2 1/3 or 1/5 of the units, with respect to SC96 amount in unit. Furthermore, the citrate consumption was twice faster than in the counterpart with SC96 (at the same pH), with a positive influence on the aromatic profile (at pH 4.50 the residual citrate was the 44% while in the blend with SC96 was left the 94%. The blend with SC226 reduced at 1/5 of the unit had only the 6% of the residual citrate) (Figure 5 and Table 5).

**Table 5:**

| | | | **Day 1, storage at 5°C-sensory analysis stirred (score 0-III maximum)** | | | | | | | |
|---|---|---|---|---|---|---|---|---|---|---|
| | **Time to reach** | **Residual citric acid (%) at pH 4,55±0,05** | **pH** | **Overall texture (s/g)** | **Ropiness** | **Body** | **Lumpiness** | **Acidity** | **Aroma** | **Gas** |
| | **pH 4,5** | | | | | | | | | |
| **Blend containing SC96** | 16h20 | 94% | 4,48 | 7,5 | III | II/III | 0 | I | II | 0 |
| **Blend containing SC226** | 16h20 | 84% | 4,49 | 13,0 | III | III | 0 | I | II | 0 |
| **Blend containing SC226, 1/2 units** | 16h45 | 44% | 4,47 | 11,5 | III | III | 0 | I | II | 0 |
| **Blend containingSC226, 1/3 units** | 15h30 | 16% | 4,38 | 12,9 | III | III | 0 | I | II | 0 |
| **Blend containingSC226, 1/5 units** | 16h40 | 5% | 4,39 | 12,7 | III | III | 0 | I | II | 0 |

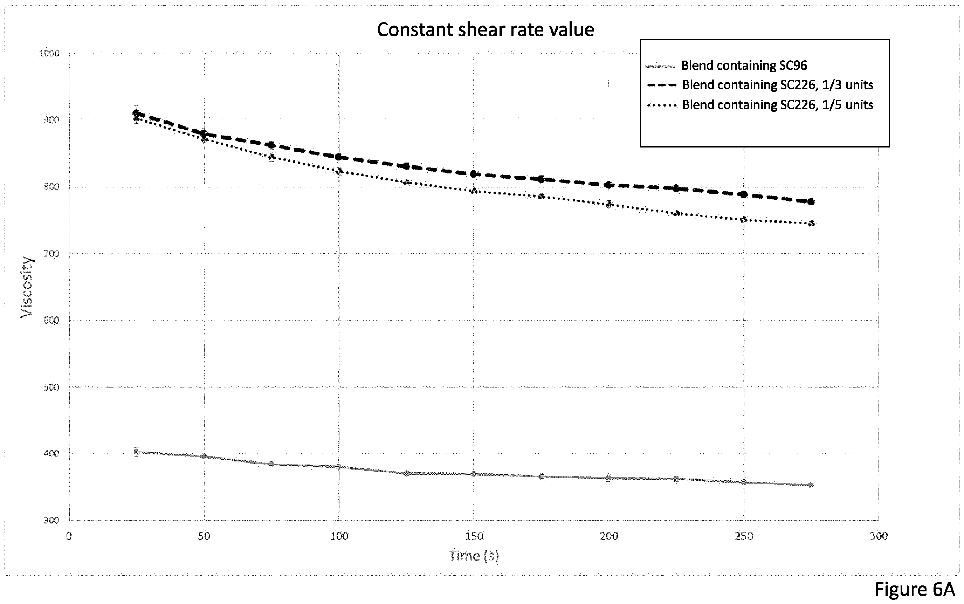

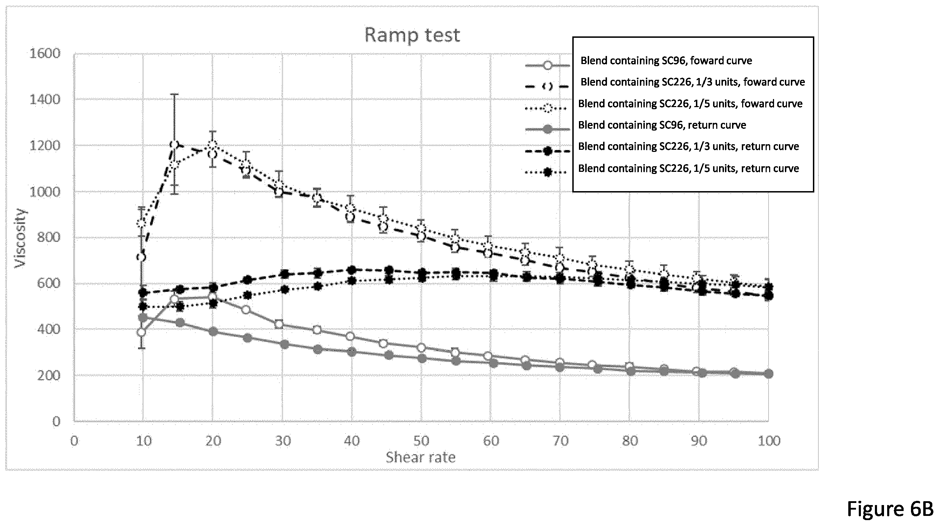

In all the set up trials, it resulted clear that half the unit amounts (or even lower) of the SC226 strain with respect to SC96, were enough to achieve a good product: the reduction of the units of inoculum does not impair the acidification performance and the yield in exopolysaccharides production is so increased with SC226 that a reduced inoculum can be applied to achieve a standard product (or even improved). At the viscosimeter were recorded higher viscosity for the blend containing SC226, even in reduced amounts (1/3 and 1//5 of the units, Figure 6). The results reported were generated using a rotational viscometer first prodig CP1000.

The analysis were conducted using the CP6020 spin (60mm diameter, 20° angle). Both the temperature of the sample and the temperature of the plate was 10°C.

Two different program were used in order to measure the viscosity of the samples. The first with an increasing value of shear rate and falling curve. The second with a constant shear rate value (30s-1) and measuring point every 25 seconds.

Prior to viscosity measurements, the yogurts were manually stirred according to a standardized protocol. Also, the time between homogenization and viscosity measurement was standardized.

Notably, the reduction of the inoculum units does not impair the acidification performance.

From the above description and the above-noted examples, the advantage attained by the strains described and obtained according to the present invention are apparent.

## Claims

1. A *Lactococcus cremoris* strain, wherein said strain is chosen from the group consisting of SC226 deposited with accession number DSM 34237 at the DSMZ and SC244 deposited with accession number DSM 34238 at the DSMZ.

2. A composition comprising one or more *Lactococcus. cremoris* strains according to claim 1.

3. The composition according to claim 2, wherein the one or more *Lactococcus cremoris* strains is freeze-dried, tyndallized, frozen pellet or in the form of a suspension.

4. The composition according to any one of claims 2 or 3, wherein the composition comprises milk, artificial milk, plant-based milk, plant-based extracts, vegetable and/or fruit matrices or juices.

5. Use of the *Lactococcus cremoris* strain according to claim 1 or the composition according to anyone of claims 2 to 4, for the preparation of a starter formulation.

6. Use of the *Lactococcus cremoris* strain according to claim 1 or the composition according to anyone of claims 2 to 4, for the preparation of a food product.

7. A food product or beverage comprising one or more *Lactococcus cremoris* strains chosen from the group consisting of: SC226 deposited with accession number DSM 34237 at the DSMZ or SC244 deposited with accession number DSM 34238 at the DSMZ.

8. The food product or beverage according to claim 7, wherein said food product is a dairy product, a milk product, a plant based product or a vegetable and/or fruit fermented product.

9. A method for preparing a dairy product, a milk product, a plant based product or a vegetal and/or fruit product, said method comprising the step of using one or more *Lactococcus cremoris* strains chosen from the group consisting of SC226 deposited with accession number DSM 34237 at the DSMZ or SC244 deposited with accession number DSM 34238 at the DSMZ.
